# EUROPEAN PATENT APPLICATION

(11) **EP 4 653 030 A1**
(43) Date of publication of application: **26.11.2025**
(21) Application number: 25174764.8
(22) Date of filing: 07.05.2025
(51) Int. Cl.: A61M 5/315

(54) **SYSTEM AND APPARATUS FOR DISPENSING PRECISE DOSAGES OF FLUID**

(30) Priority: 19.05.2024 US 202418668237
(71) Applicant: Sunshine Enclosures LLC, New York, NY 10016 (US)
(72) Inventor: UBELL, Edward, New York (US); RUSSELL, Ryan, New York (US); KIRSH, Ross, Florida (US)
(74) Representative: IK-IP LTD

(57) **Abstract**

A fluid dispenser comprising: a housing; a cartridge enclosing a fluid; a scroll wheel comprising: a first portion disposed within an interior of the housing, a second portion partially protruding from the housing, and scroll wheel interior teeth; a first gear comprising first gear exterior teeth, the first gear disposed within the interior of the housing, the first gear exterior teeth in mechanical communication with the scroll wheel interior teeth; a plunger comprising plunger exterior teeth in mechanical communication with the first gear exterior teeth, the plunger in fluid communication with the cartridge, the plunger further comprising a plunger slot, wherein the scroll wheel traverses the plunger slot, wherein the plunger is configured to move within the housing and the cartridge, wherein the plunger is configured to compress the fluid, wherein the compression of fluid is configured to drive the fluid from a cartridge trip disposed atop the cartridge.

## Description

### FIELD OF THE INVENTION

The present disclosure is directed to a system and apparatus for dispensing fluids. Specifically, the present disclosure is directed to a system and apparatus for dispensing precise dosages of fluids.

### INTRODUCTION

Fluid dispensers, an indispensable cornerstone of modern medical practice, hold a pivotal role in the meticulous administration of fluids-a critical aspect within the medical domain. The precision in dosing, paramount to patient care, ensures that individuals receive the precise and essential quantity of fluids. Traditionally, healthcare providers and patients have turned to the tried-and-true syringe for the accurate delivery of fluids and liquid medications. Despite their precision, syringes often elicit feelings of intimidation and reluctance among patients.

In response to the apprehension associated with syringes, a wave of innovative solutions has surfaced, aspiring to offer patients a more approachable method for fluid dispensing. However, these alternatives, while well-intentioned, come with their own unique set of challenges. Some prove cumbersome, introducing complexities into the fluid dispensing process. Meanwhile, others grapple with providing the seamless convenience of single-handed operation. To illustrate, individuals grappling with hand pain or arthritis find themselves unable to employ existing fluid dispensers with a single hand, presenting a distinct barrier to use.

The ongoing pursuit of user-friendly and efficient alternatives to traditional syringes persists, motivated by the imperative to elevate the patient experience in the realm of medical fluid administration. An essential consideration in this quest revolves around economic usage, especially for patients experiencing pain. Consequently, a fluid dispenser designed for single-handed use holds significant promise. Furthermore, the aspiration extends beyond mere functionality; it is desirable to introduce a fluid dispenser that not only aligns with the ergonomic needs of medical patients but also sidesteps the stigma typically associated with conventional medical syringes.

Additionally, many conventional fluid dispensing devices suffer from imprecise dosing or product waste due to the propensity for convention devices to "leak" or protrude fluid in excess of the desired quantity. For example, many manually actuated devices may extrude more fluid that desired as a function of the momentum of the fluid itself. In such conventional fluid dispensing devices, manual actuation (e.g., compression of a syringe plunger) may cause the fluid to exit the outlet even after manual actuation is ceased as a function of the momentum of the fluid.

Even more ideally, the envisioned product should transcend the conventional expectations, seamlessly integrating into the daily lives of medical patients. It should not only accommodate single-handed operation but also contribute to destigmatizing the fluid dispensing process. This pursuit culminates in the conceptualization of a groundbreaking product, capable of revolutionizing the landscape of fluid administration. The disclosure of such an innovative solution is presented herein, charting a course toward a future where patient-centricity and ease of use converge in a singular, transformative fluid dispenser.

### SUMMARY

This Summary is provided to introduce a selection of concepts in a simplified form that are further described below in the Detailed Description. This Summary is not intended to identify key features or essential features, nor is it intended to limit the scope of the claims included herewith.

Provided may be a fluid dispenser comprised of a housing; a cartridge enclosing a fluid; a scroll wheel comprising: a first portion disposed within an interior of the housing, a second portion partially protruding from the housing, and scroll wheel interior teeth; a first gear comprising first gear exterior teeth, the first gear disposed within the interior of the housing, the first gear exterior teeth in mechanical communication with the scroll wheel interior teeth; a plunger comprising plunger exterior teeth in mechanical communication with the first gear exterior teeth, the plunger in fluid communication with the cartridge, the plunger further comprising a plunger slot, wherein the scroll wheel traverses the plunger slot, wherein the plunger is configured to move within the housing and the cartridge, wherein the plunger is configured to compress the fluid, wherein the compression of fluid is configured to drive the fluid from a cartridge trip disposed atop the cartridge.

**In** an embodiment, the housing is further comprised of a first housing piece, having a first notch, and a second housing piece, having a second notch, the first notch and the second notch forming a housing aperture.

**In** a further embodiment, the fluid dispenser further comprises a lock disposed upon the housing, the lock in fluid communication with the interior of the housing.

In yet a further embodiment, the fluid dispenser further comprises a dispenser cap reversibly coupled to the housing, such that the fluid dispenser may be in at least one of an open configuration and a closed configuration.

In an additional embodiment, rotational movement of the scroll wheel in a predetermined increment produces a feedback stimulus. Further, the rotational movement of the scroll wheel in the predetermined increment compresses a predetermined volume of the fluid. Moreover, the predetermined increment is 60 degrees. Additionally, the predetermined volume is 0.05mL. I

In another embodiment, the rotational movement of the scroll wheel is translated into a rotational movement of the first gear, the rotational movement of the first gear is translated into a linear movement of the plunger, and the linear motion of the plunger compresses the predetermined volume of the fluid.

Provided may be a fluid dispenser comprised of a housing; a cartridge enclosing a fluid; a scroll wheel comprising: a first portion disposed within an interior of the housing, a second portion partially protruding from the housing, scroll wheel interior teeth, and scroll wheel exterior teeth; a first gear comprising first gear exterior teeth, the first gear disposed within the interior of the housing, the first gear exterior teeth in mechanical communication with the scroll wheel interior teeth; a second gear comprising a first set of second gear teeth and a second set of second gear teeth, the second gear disposed within the interior of the housing, the first set of second gear teeth in mechanical communication with the first gear exterior teeth; a plunger comprising plunger exterior teeth in mechanical communication with the second set of second gear teeth, the plunger in fluid communication with the cartridge, the plunger further comprising a plunger slot, wherein the scroll wheel traverses the plunger slot, wherein the plunger is configured to move within the housing and the cartridge, wherein the plunger is configured to compress the fluid, wherein the compression of fluid is configured to drive the fluid from a cartridge trip disposed atop the cartridge.

In an embodiment, the second gear is a two-step gear. Additionally, the fluid dispenser further comprises a roller disposed adjacent to the plunger. Furthermore, the fluid dispenser further comprises a slot guide disposed within a lower portion of the plunger. In addition, the fluid dispenser further comprised of a post captured by the slot guide.

Provided may be a fluid dispenser comprised of a housing; a cartridge enclosing a fluid; a scroll wheel comprising: a wheel base comprising: a first portion disposed within an interior of the housing, and a second portion partially protruding from the housing, and at least one wheel gear disposed upon the wheel base; one or more plunger gears in mechanical communication with the at least one wheel gear; and a plunger in mechanical communication with the one or more plunger gears, wherein the plunger is configured to move within the housing and the cartridge, wherein the plunger is configured to compress the fluid, wherein the compression of fluid is configured to drive the fluid from a cartridge trip disposed atop the cartridge.

In another embodiment, the at least one wheel gear is further comprised of a first wheel gear disposed on a first side of the wheel base, and a second wheel gear disposed on a second side of the wheel base.

In an embodiment, the one or more plunger gears are further comprised of a first plunger gear comprising: a first primary gear in mechanical communication with the first wheel gear, and a first secondary gear disposed upon the first primary gear; and a second plunger gear comprising: a second primary gear in mechanical communication with the second wheel gear, and a second secondary gear disposed upon the second primary gear.

In a further embodiment, the plunger is further comprised of a plurality of plunger teeth disposed within a gear slot, the plurality of plunger teeth in mechanical communication with at least one of the first secondary gear and the second secondary gear; and a plunger tip disposed upon a distal end of the plunger, the plunger tip configured to compress the fluid, the plunger tip proscribed from entering the interior of the housing.

In yet a further embodiment, a rotational movement of the scroll wheel is translated into a rotational movement of the first wheel gear and the second wheel gear, the rotational movement of the first wheel gear and the second wheel gear is translated into a rotational movement of the first primary gear, the first secondary gear, the second primary gear, and the second secondary gear, the rotational movement of the first primary gear, the first secondary gear, the second primary gear, and the second secondary gear, is translated into a liner movement of the plunger tip, and the linear movement of the plunger tip compresses the predetermined volume of the fluid.

### BRIEF DESCRIPTION OF THE DRAWINGS

The incorporated drawings, which are incorporated in and constitute a part of this specification exemplify the aspects of the present disclosure and, together with the description, explain and illustrate principles of this disclosure.
FIG. 1 is a sectional view of an embodiment of an apparatus for dispensing precise dosages of fluid.
FIG. 2 is an interior view of an embodiment of the apparatus for dispensing precise dosages of fluid.
FIG. 3 is an illustration of an embodiment of the gear mechanism of the apparatus for dispensing precise dosages of fluid.
FIG. 4 is a sectional view of an embodiment of the gear mechanism of the apparatus for dispensing precise dosages of fluid in a drained state.
FIG. 5 is an illustration of an embodiment of the gear mechanism of the apparatus for dispensing precise dosages of fluid in the drained state.
FIG. 6 is a sectional view of an embodiment of the gear mechanism of the apparatus for dispensing precise dosages of fluid in an undrained state.
FIG. 7 is an illustration of an embodiment of the gear mechanism of the apparatus for dispensing precise dosages of fluid in an undrained state.
FIG. 8 is an illustration of an embodiment of one or more plunger gears.
FIG. 9 is an illustration of an embodiment of a first scroll wheel side.
FIG. 10 is an illustration of an embodiment of a second scroll wheel side.
FIG. 11 is an illustration of an embodiment of a plunger.
FIG. 12 is an illustration of an embodiment of the apparatus for dispensing precise dosages of fluid.
FIG. 13 is an illustration of an embodiment of a cross-sectional view of a cartridge.

### DETAILED DESCRIPTION

In the following detailed description, reference will be made to the accompanying drawing(s), in which identical functional elements are designated with like numerals. The aforementioned accompanying drawings show by way of illustration, and not by way of limitation, specific aspects, and implementations consistent with principles of this disclosure. These implementations are described in sufficient detail to enable those skilled in the art to practice the disclosure and it is to be understood that other implementations may be utilized and that structural changes and/or substitutions of various elements may be made without departing from the scope and spirit of this disclosure. The following detailed description is, therefore, not to be construed in a limited sense.

It is noted that description herein is not intended as an extensive overview, and as such, concepts may be simplified in the interests of clarity and brevity.

All documents mentioned in this application are hereby incorporated by reference in their entirety. Any process described in this application may be performed in any order and may omit any of the steps in the process. Processes may also be combined with other processes or steps of other processes.

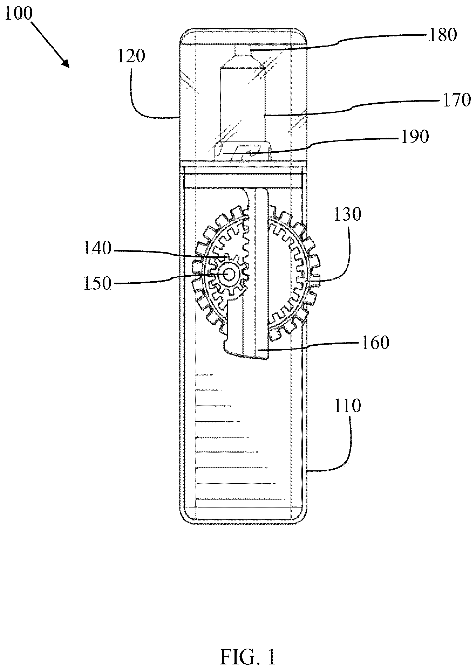

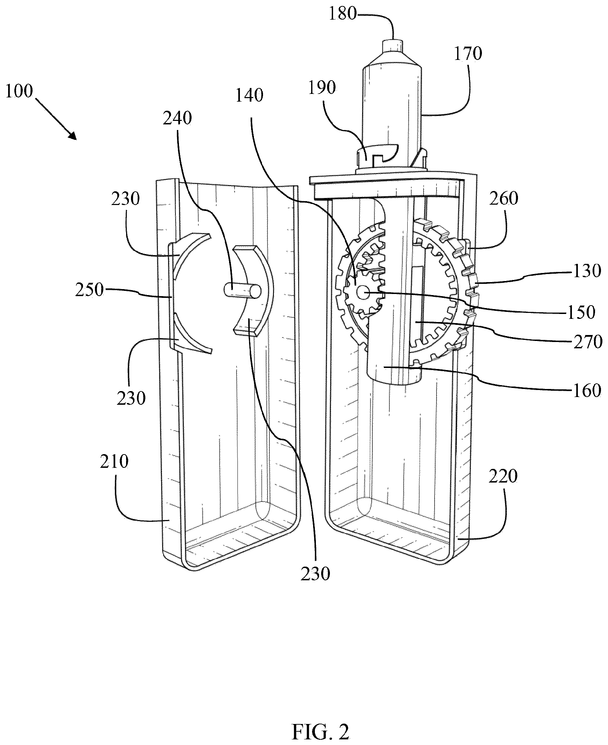

Starting with FIGS. 1 and 2, the system and apparatus for dispensing precise dosages of fluid (hereinafter the "fluid dispenser") 100 may be comprised of at least a housing 110 and a dispenser cap 120. In an embodiment, the dispenser cap 120 may be reversibly coupled to the housing 110, such that the fluid dispenser 100 may be in at least one of an open configuration and a closed configuration. Said fluid dispenser 100, in the closed configuration, may be child-proof or child-resistant, such that children below a specific age experience difficulty or are unable to transpose the fluid dispenser 100 from the closed configuration to the open configuration. As a nonlimiting example, a user may apply a force to the dispenser cap 120, while the fluid dispenser 100 is in the closed configuration, wherein said force is capable of removing the cap 120 from the housing 110, thus transposing the fluid dispenser from the closed configuration to the open configuration. The fluid dispenser 100 may be comprised of one or more materials. In an embodiment, the fluid dispenser 100 is comprised of polychlorotrifluoroethylene (PCTFE) and/or borosilicate glass. Further, the fluid dispenser 100 may be configured to be handled and/or manipulated by a user. In an embodiment, the user is able to utilize and manipulate the fluid dispenser 100 with one hand. In one embodiment, the dispenser cap 120 may be reversibly coupled to the housing 110 with a fastening means that requires two hands to separate, while operation of the scroll wheel 130 may be conducted with one hand. In such an embodiment, access to the cartridge tip 180 may be thwarted by the child resistant nature of the dispenser cap 120, while fluid may be dispensed with a single hand (e.g., a user's thumb) via the scroll wheel 130 once the dispenser cap 120 child resistant feature has been overcome.

The housing 110 may be comprised of at least a first housing piece 210 and a second housing piece 220, wherein the first housing piece 210 is coupled (e.g., reversibly coupled) to the second housing piece 220, or *vice versa.* In an alternate embodiment, the first housing piece 210 and the second housing piece 220 may be decoupled for reloading fluid in the device. The first housing piece 210 and the second housing piece 220 may configured such that separation of such components is not actionable by a standard user, for example, in an instance where the fluid dispenser 100 is of a disposable or one-time-use nature. Accordingly, in such an instance, the first housing piece 210 and the second housing piece 220 may be permanently adhered to one another (e.g., via commercial adhesive or another permanent fastening means).

The housing 110 may be in at least one of an open configuration and a closed configuration. As a nonlimiting example, FIG. 1 depicts the housing 110 in a closed configuration, and FIG. 2 depicts the housing 110 in an open configuration. Further, the first housing piece 210 and the second housing piece 220 may incorporate a first notch 250 and/or a second notch 260. In a further embodiment, the first housing piece 210 may incorporate the first notch 250 and the second housing piece 220 may incorporate the second notch 260, or *vice versa.* In yet a further embodiment, the first notch 250 and the second notch 260 may form a housing aperture 135 (e.g., configured to partially house the scroll wheel 130, described below) while the housing 110 is in the closed configuration. In such an embodiment, the interior of the housing 110 may be in fluid communication with the outside environment via the housing aperture 135.

The fluid dispenser 100 may be further comprised of a scroll wheel 130, a first gear 140, and/or a plunger 160, wherein said first gear 140 may incorporate a first gear aperture 150. In an embodiment, the scroll wheel 130, the first gear 140, and/or the plunger 160 may be disposed within an interior of the housing 110. In such an embodiment, at least one of the scroll wheel 130, the first gear 140, and the plunger 160 may be enclosed within the housing 110 while said housing 110 is in the closed configuration. In an embodiment, the scroll wheel 130, the first gear 140, and/or the plunger 160 may be exposed to an outside environment while the housing 110 is in the open configuration. In an alternative embodiment a first portion of at least one of the scroll wheel 130, the first gear 140, and the plunger 160 may be exposed to the outside environment while a second portion of at least one of the scroll wheel 130, the first gear 140, and the plunger 160 may be enclosed within the housing 110. In such an embodiment, the scroll wheel 130, the first gear 140, and/or the plunger 160 may traverse the housing aperture 135, thus enabling the first portion to be exposed and the second portion to be enclosed. As a nonlimiting example, the first portion of the scroll wheel 130, the first gear 140, and the plunger 160 may be enclosed within the housing 110 while the second portion of the scroll wheel 130 traverses the housing aperture 135 and is thus exposed to the outside environment. In such a nonlimiting example, no more than 10% of an area of the second portion of the scroll wheel 130 may traverse the housing aperture 135. In another nonlimiting example, no more than 20% of an area of the second portion of the scroll wheel 130 may traverse the housing aperture 135. In yet another nonlimiting example, no more than 30% of an area of the second portion of the scroll wheel 130 may traverse the housing aperture 135.

Further, the scroll wheel 130 may be comprised of a plurality of teeth, wherein said teeth may be disposed upon at least one of an interior surface and an exterior surface of the scroll wheel 130. In an embodiment, the first gear 140 may be comprised of a plurality of teeth, wherein said teeth may be disposed upon an interior surface and/or an exterior surface of the first gear 140. In another embodiment, the plunger 160 may be comprised of a plurality of teeth, wherein said teeth are disposed upon an exterior surface of the plunger 160. Thus, as depicted in FIG. 2, the fluid dispenser 100 may include scroll wheel exterior teeth, scroll wheel interior teeth, first gear exterior teeth, and plunger exterior teeth.

In an embodiment, the scroll wheel 130 may be in mechanical communication with the first gear 140 and/or the plunger 160. Accordingly, the plurality of teeth of the scroll wheel 130, for example the scroll wheel interior teeth, may be in mechanical communication with the plurality of teeth of at least one of the first gear 140, for example the first gear exterior teeth, and, thus, indirectly capable of driving the plunger 160, for example, via the plunger exterior teeth. In an embodiment, the first gear 140 may be in mechanical communication with the scroll wheel 130 and/or the plunger 160. In such an embodiment, the first great exterior teeth of the first gear 140 may be in mechanical communication with the scroll wheel interior teeth of the scroll wheel 130 and/or the plunger exterior teeth of the plunger 160. In a further embodiment, the plunger 160 may incorporate a scroll wheel slot 270. In yet a further embodiment, the scroll wheel 130 may be disposed within the scroll wheel slot 270, such that the scroll wheel 130 may extend through the plunger 160. Further, the first gear 140 may be disposed between the interior surface of the scroll wheel 130 and the plunger 160. In such an embodiment, the scroll wheel 130 may be disposed within the scroll wheel slot 270.

Additionally, movement of the scroll wheel 130 may translate to movement of the first gear 140 and/or the plunger 160. Moreover, movement of the first gear 140 may translate to movement of the scroll wheel 130 and/or the plunger 160. In an embodiment, rotational movement of the scroll wheel 130 may translate to rotational movement of the first gear 140. In such an embodiment, rotational movement of the scroll wheel 130 may be user actuated. Rotational movement of the first gear 140 may translate to movement of the plunger 160. In such an embodiment, the plunger 160 movement may be linear movement. As a nonlimiting example, the scroll wheel 130, traversing both the housing aperture 135 and the plunger 160, may be rotationally moved via user actuation of at least one of the first portion and the second portion of the scroll wheel 130. In some instances, the "second portion" of a component may be defined as the portion of said component that extends externally from the housing aperture 135. In such a nonlimiting example, the rotational movement of the scroll wheel 130 translates to rotational movement of the first gear 140, said first gear 140 being disposed between the interior surface of the scroll wheel 130 and the plunger 160. In continuance of the aforementioned example, the rotational movement of the first gear 140 may translate to linear movement of the plunger 160. As another nonlimiting example, the first gear 140 and the plunger 160 may act as a rack and pinion system, wherein the first gear 140 is the pinion and the plunger 160 is the rack. In such an example, rotational movement of the scroll wheel 130, via user actuation of at least one of the first portion and the second portion of said scroll wheel 130, is translated to rotational movement of the first gear 140, acting as the pinion, which in turn is translated to linear movement of the plunger 160, acting as the rack. Moreover, the utilization of three geared components (e.g., the scroll wheel 130, the first gear 140, and the plunger 160) allow the fluid to eject forward based on forward rotation of the scroll wheel 130 according to the user's point of view. Similarly, backward rotation of the scroll wheel 130 from the user's perspective may cause the fluid to withdraw into the device.

Further, user actuation of the scroll wheel 130 may produce a user feedback stimulus for every predetermined increment of scroll wheel 130 movement. In an embodiment, said feedback stimulus may be at least one of an auditory stimulus, a haptic stimulus, and a visual stimulus. In an embodiment, the user feedback stimulus may be a combination of two or more of the auditory stimulus, the haptic stimulus, and/or the visual stimulus. As a nonlimiting example, the user feedback stimulus may be an auditory and/or haptic "click," thus notifying said user the scroll wheel 130 has been moved one predetermined increment. Moreover, one predetermined increment may equate to a distance the scroll wheel 130 has moved. In an embodiment, user actuation of the scroll wheel 130 may produce the user feedback stimulus for every 60 degrees the scroll wheel 130 rotationally moved. However, the user feedback stimulus may occur for any degree of scroll wheel rotational movement between 1 and 360 degrees. However, in instances where a desired discrete volume of fluid translates to more than a full rotation of the scroll wheel 130, the scroll wheel 130 may provide user feedback stimulus in increments beyond 360 degrees. In an embodiment, the feedback stimulus of the scroll wheel 130 is correlated to a non-arbitrary predetermined volume of fluid. For example, the scroll wheel 130 may be sized and configured such that a certain number of increment of feedback stimulus release a certain desired non-arbitrary predetermined volume of fluid. Thus, the feedback stimulus may inform the user as to the volume of fluid being ejected. Accordingly, such a feedback stimulus may allow a user to dose (or otherwise release a desired volume of fluid) based exclusively on the feedback stimulus.

The fluid dispenser 100 may be further comprised of a scroll wheel housing 230 and a gear pin 240. In an embodiment, the scroll wheel housing 230 and/or the gear pin 240 may be disposed upon an interior surface of at least one of the first housing piece 210 and the second housing piece 220. In another embodiment, the scroll wheel housing 230 may act as a support for the scroll wheel 130. In such an embodiment, the scroll wheel housing 230 may allow the scroll wheel 130 to move about a defined position, thus ensuring the scroll wheel 130 is not dislodged from said defined position during user actuation. Moreover, the scroll wheel housing 230 may include a voided section allowing the scroll wheel housing 230 to at least partially surround the scroll wheel 130 while also accommodating the space occupied by the plunger 160. In a further embodiment, the gear pin 240 may act as a support for the first gear 140. In yet a further embodiment, the gear pin 240 may be configured to traverse at least one of the first gear 140 and the first gear aperture 150. In such an embodiment, the gear pin 240 may allow for the first gear 140 to rotate about a defined position, thus ensuring the first gear 140 is not dislodged from said defined position during user actuation of the scroll wheel 130.

The fluid dispenser 100 may be further comprised of a lock 190. In an embodiment, the lock 190 may be disposed upon at least one of the first housing piece 210 and the second housing piece 220. In such an embodiment, the dispenser cap 120 may be configured to cover the lock 190 in the closed configuration. In another embodiment, the plunger 160 may be in fluid communication with the lock 190, such that the plunger 160 may extend through the lock 190. In such an embodiment, movement of the plunger 160 may enable said plunger 160 to move from the interior of the housing 110 to the outside environment. In a nonlimiting example, the plunger 160 may, via linear movement, move from the interior of the housing 110 to the outside environment by extending through the lock 190. In some embodiments, the plunger 160 may be capable of extending at least partially above the lock 190, wherein actuation of the scroll wheel 130 may allow the plunger to move vertically relative to the lock 190. The lock 190 may be configured to interface with the cap 120, allowing the cap 120 and lock 190 to form a child resistant coupling.

The fluid dispenser 100 may be further comprised of a cartridge 170, wherein said cartridge 170 may incorporate a cartridge tip 180. The cartridge tip may have a diameter between 1mm and 5mm. Additionally, the cartridge 170 may be comprised of glass, and may enclose a fluid. In an embodiment, the fluid may be a cannabis derived oil. In an embodiment, the cannabis derived oil may be Rick Simpson Oil (RSO). However, the cartridge 170 may enclose any suitable fluid alternative (e.g., honey, soap, water, etc.). Further, the cartridge 170 may be in fluid communication with the cartridge tip 180, such that the fluid may pass from the cartridge 170, through the cartridge tip 180, to the outside environment. In another embodiment, the cartridge 170 may be reversibly coupled to the lock 190, such that the cartridge 170 may be in at least one of a locked configuration and an unlocked configuration. In such an embodiment, the user may apply a force to the cartridge 170 and/or the lock 190, thus transposing the cartridge 170 from the locked configuration to the unlocked configuration, or *vice versa.* In an embodiment, the force may be user actuated rotational movement of the cartridge 170 and/or the lock 190. Alternatively, the cartridge 170 may be permanently affixed to the lock 190. In such an embodiment, the dispenser cap 120 may be configured to cover the cartridge 170 and/or the lock 190 in the closed configuration. The dispenser cap 120 may include complementary geometry to the lock 190, such that manipulation of the dispenser cap 120 causes the dispenser cap 120 to unlock from the lock 190.

The cartridge 170 may be further comprised of a cartridge opening (not shown), wherein said opening may enable fluid communication between the cartridge 170 and the lock 190 while in the locked configuration. In such an embodiment, the plunger 160 may be in fluid communication with the cartridge 170. As a nonlimiting example, movement of the plunger 160, may force the fluid, enclosed by the cartridge 170, through the cartridge tip 180, to the outside environment. As another nonlimiting example, movement of the plunger 160 may prevent the fluid from exiting the cartridge into the outside environment. For example, braking the scroll wheel 130 may prevent movement of the plunger 160 and, therefore, may be utilized by a user to cease flow of fluid from cartridge tip 180. Such a braking method may be used even when the fluid flow is involuntary, for example in an instance where the fluid exiting the dispenser 100 is due to the inertial movement of the fluid. In a further embodiment, the cartridge opening may have a diameter between 10mm and 20mm. In yet a further embodiment, the cartridge 170 may have a length between 10mm and 30mm. Moreover, the cartridge may have a volume between 0.5mL and 1.5mL. However, the cartridge 170 may have any suitable opening area, length, or volume.

Further, movement of the scroll wheel 130 one predetermined increment may dispense a predetermined volume of the fluid. In an embodiment, movement of the scroll wheel 130 one predetermined increment may dispense 0.05mL of the fluid. However, any suitable volume of fluid between 0.01mL and 0.1mL may be dispensed for every one predetermined increment of scroll wheel 130 movement. As a nonlimiting example, user actuation of the scroll wheel 130, producing movement of the scroll wheel 130 one predetermined increment, may produce linear movement of the plunger 160, thus dispensing one predetermined volume of the fluid. In a further embodiment, user actuation of the scroll wheel 130 counterclockwise may result in at least one of the fluid being dispensed and the fluid being retained. In yet a further embodiment, user actuation of the scroll wheel 130 clockwise may result in at least one of the fluid being dispensed and the fluid being retained.

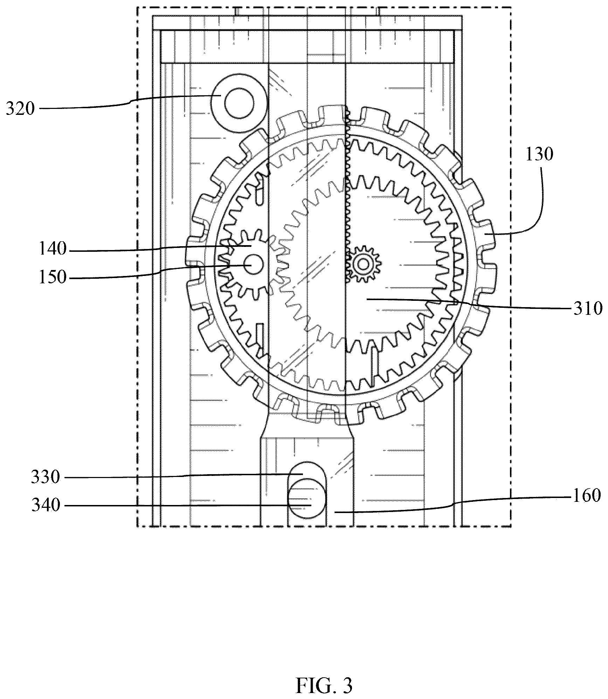

Referring to FIG. 3, the fluid dispenser 100 may be further comprised of a second gear 310. Further, the second gear may be in mechanical communication with at least one of the scroll wheel 130, the first gear 140, and the plunger 160. In an embodiment, the second gear 310 is comprised of a plurality of teeth, wherein said teeth are disposed upon an outside surface of the second gear 310. In such an embodiment, the plurality of teeth of the second gear 310 may be in mechanical communication with the plurality of teeth of at least one of the scroll wheel 130, the first gear 140, and the plunger 160. The second gear 310 may be disposed between the interior surface of the scroll wheel 130 and the plunger 160. As a nonlimiting example, the first gear 140 and the second gear 310 are disposed upon an interior surface of the scroll wheel 130. In such a nonlimiting example, movement of the scroll wheel 130, translates to movement of the first gear 140, which translates to movement of the second ger 310, resulting in movement of the plunger 160. In an embodiment, the movement of the second gear is rotational movement. In another embodiment, the second gear 310 may be a two-step gear. As a nonlimiting example, the second gear 310 and the plunger 160 may act as the rack and pinion system, wherein the second gear 310 is the pinion and the plunger 160 is the rack. In such an example, rotational movement of the scroll wheel 130, via user actuation of at least one of the first portion and the second portion of said wheel 130, is translated to rotational movement of the first gear 140, which is translated into rotational movement of the second gear 310, acting as the pinion, which in turn is translated to linear movement of the plunger 160, acting as the rack, wherein said linear movement of the plunger 160 results in the fluid being dispensed into the outside environment.

The second gear 310 may increase a gear ratio. In an embodiment, the increase in the gear ratio may increase user leverage, thus allowing the user to more easily actuate the scroll wheel 130 and/or more easily dispense a given volume of fluid. As a nonlimiting example, the user may actuate the scroll wheel 130, thus resulting in rotational movement of the scroll wheel 130 in the amount of 60 degrees. The rotational movement of the scroll wheel 130 results in rotational movement of the first gear 140, which is translated into rotational movement of the second gear 310. The rotational movement of the second gear 310 is translated into linear movement of the plunger 160, which in turn results in 0.05mL of the fluid being dispensed into the outside environment.

In an embodiment, the second gear 310 may include a first set of second gear teeth and a second set of second gear teeth. In an embodiment, the first gear exterior teeth may interface with the first set of second gear teeth, causing the entire second gear 310 to rotate (e.g., including the second set of gear teeth), further causing the plunger 160 to drive. The first set of second gear teeth may have a greater diameter than the second set of second gear teeth.

In an embodiment, the housing 110 may include a roller 320 disposed adjacent to the plunger 160 and opposite of the rotating direction to ensure the plunger 160 does not bend during plunger 160 movement. The roller 320 may be a cylindrical member and may be configured to freely spin about an axis. Thus, the plunger 160 may ride along the roller 320, mitigating unwanted flex in the plunger, while not inhibiting the desired motion of the plunger 160.

In an embodiment, the plunger 160 includes a slot guide 330. The slot guide 330 may be a cavity in the lower portion of the plunger 160. The slot guide 330 may be configured to capture a post 340. The post 340 may be affixed to the housing 110. Accordingly, the maximum extension and maximum retraction of the plunger 160 may be controlled by the interfacing of the post 340 with either side of the slot guide 330. Moreover, the plunger 160 may be stabilized by the post 340 within the slot guide 330. As a function of the flexibility of the plunger 160 and/or the pressure or the resistance felt by the plunger during operation, the plunger 160, in imperfect conditions, may flex. Thus, the interfacing of the slot guide 330 with the post 340 may maintain the vertical positioning of the plunger 160.

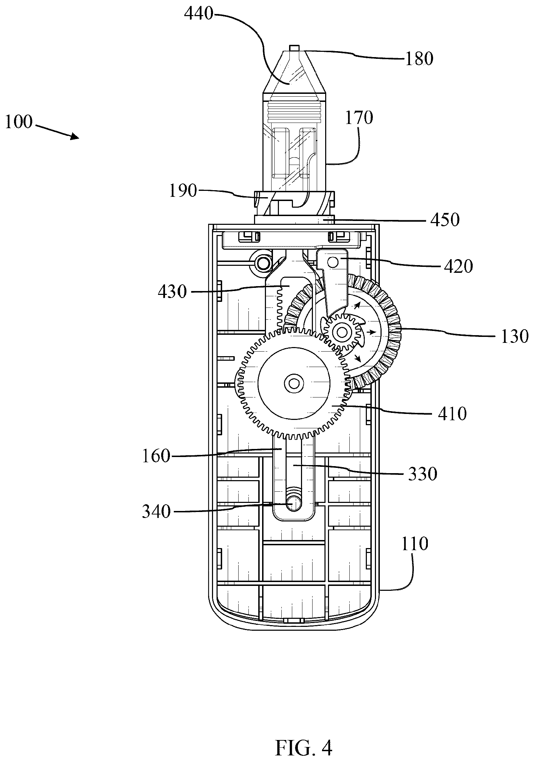

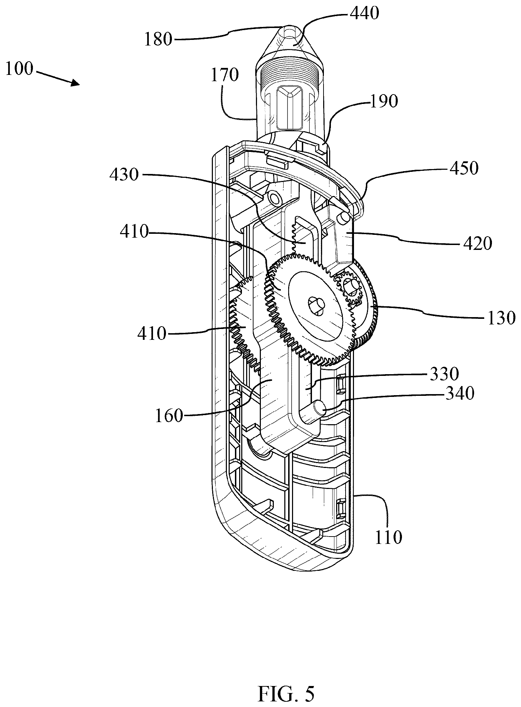

Turning to FIGS. 4 and 5, the fluid dispenser may be further comprised of one or more plunger gears 410, a click clutch 420, a dispenser needle 440, and a housing cap 450. In an embodiment, the dispenser needle 440 may be disposed within an interior of the cartridge 170. In such an embodiment, the dispenser needle 440 may be configured to direct the fluid from the cartridge 170 through the cartridge tip 180 to the outside environment.

Moreover, the housing cap 450 may be disposed atop the housing 110. In an embodiment, the housing cap 450 may be configured to facilitate coupling between the first housing piece 210 and the second housing piece 220. In such an embodiment, the housing cap 450 may be comprised of a plurality of recesses, wherein a plurality of protrusions upon at least one of the first housing piece 210 and the second housing piece 220 are configured to traverse said recesses and reversibly couple to the housing cap 450. In another embodiment, a first portion of the housing cap 450 may be exposed to the outside environment, while a second portion of said cap 450 may be exposed to the interior of the housing 110, or *vice versa.* In an additional embodiment, the lock 190 may be disposed atop the housing cap 450, such that the lock 190 is exposed to the outside environment. Further, the housing cap 450 may facilitate straightforward deconstruction of the fluid dispenser 100. In an embodiment, the user may uncouple at least one of the first housing piece 210 and the second housing piece 220 from the housing cap 450 such that said user may access the interior of the housing 110. In such an embodiment, the user may clean the components residing within the interior of the housing 110.

In an embodiment, the one or more plunger gears 410 may be in mechanical communication with at least one of the scroll wheel 130, the first gear 140, the plunger 160, and the second gear 310. In such an embodiment, the one or more plunger gears 410 may be disposed within an interior of the housing 110. As a nonlimiting example, the one or more plunger gears 410 may be comprised of a first and second plunger gear, wherein said gears are disposed upon at least one of the first housing piece 210 and the second housing piece 220. In such an example, the plunger 160 may be further comprised of a gear slot 430, wherein a portion of the first and second plunger gears may reside.

In an embodiment, user actuated rotational movement of the scroll wheel 130 may translate to rotational movement of the one or more plunger gears 410. Additionally, rotational movement of the one or more plunger gears 410 may translate to movement of the plunger 160. In such an embodiment, the plunger 160 movement may be linear movement. As a nonlimiting example, the scroll wheel 130, traversing both the housing aperture 135 and the plunger 160, may be rotationally moved via user actuation of at least one of the first portion and the second portion of the scroll wheel 130. In such a nonlimiting example, the rotational movement of the scroll wheel 130 translates to rotational movement of both the first plunger gear and the second plunger gear, wherein said gears are partially disposed within the gear slot 430. In continuance of the aforementioned example, the rotational movement of the first and second plunger gears may translate to linear movement of the plunger 160. Moreover, the one or more plunger gears 410 and the plunger 160 may act as a rack and pinion system, wherein said gears 410 are the pinion and the plunger 160 is the rack. For example, rotational movement of the scroll wheel 130, via user actuation of at least one of the first portion and the second portion of said scroll wheel 130, is translated to rotational movement of the one or more plunger gears 410, acting as the pinion, which in turn is translated to linear movement of the plunger 160, acting as the rack. Utilization of the multi-geared system (the scroll wheel 130, the one or more plunger gears 410, and the plunger 160) allow the fluid to eject forward based on forward rotation of the scroll wheel 130 according to the user's point of view. Similarly, backward rotation of the scroll wheel 130 from the user's perspective may cause the fluid to withdraw into the device.

In a further embodiment, forward rotation of the scroll wheel 130 may be proscribed upon full depletion of the fluid residing within the cartridge 170. For example, the user, via forward rotation of the scroll wheel 130, may dispense an entirety of the fluid within the cartridge, and upon the entirety of the fluid being dispensed, forward rotation of the scroll wheel 130 may be proscribed. In such an example, the maximum extension of the plunger 160 may correspond to the entirety of the fluid being dispensed from the cartridge 170, wherein the fluid dispenser 100 may be in a drained state (as depicted in FIGS. 4 and 5). The drained state may be characterized by the absence of fluid within the cartridge 170.

The click clutch 420 may be disposed within the interior of the housing 110. In an embodiment, the click clutch 420 may be in mechanical communication with the scroll wheel 130. For example, user actuation of the scroll wheel 130 may produce the user feedback stimulus, via the click clutch 420, for every predetermined increment of scroll wheel 130 movement. In another embodiment, a proximal portion of the click clutch 420 may pivot about a pin disposed within the interior of the housing 110, while a distal end of the click clutch 420 may be in mechanical communication with the scroll wheel 130. In such an embodiment, the distal portion of the click clutch 420 may produce the auditory and haptic "click" for every one predetermined increment the user actuated the scroll wheel 130. The predetermined increment may be defined by a length of one of a plurality of clutch actuation members 930 (described in more detail below).

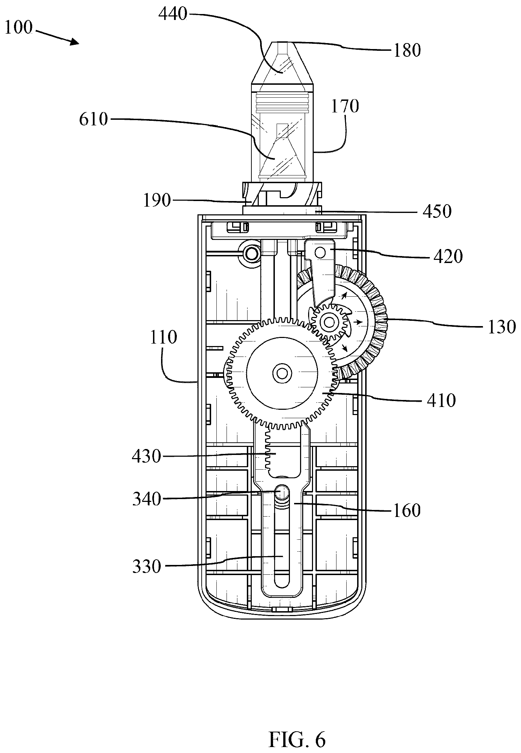

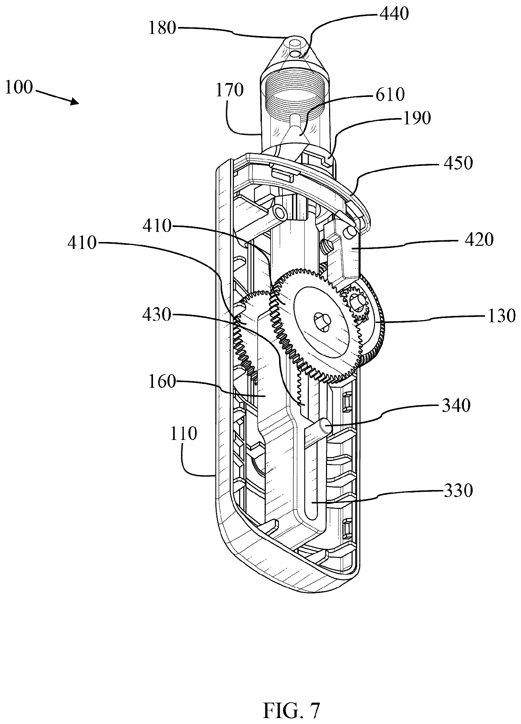

FIGS. 6 and 7 illustrate embodiments of the gear mechanism of the fluid dispenser 100 while in an undrained state. In an embodiment, the undrained state may result from maximum retraction of the plunger 160 facilitated by the interfacing of the post 340 with either side of the slot guide 330. The undrained state may be characterized by the presence of fluid within the cartridge 170. In the undrained state, a plunger tip 610 may be visible within the cartridge 170. In an embodiment, the plunger tip 610 may be disposed upon at least one of a distal end 1120 and proximal end 1130 of the plunger 160. In such an embodiment, the plunger tip 610 may be in fluid communication with at least one of the cartridge 170, the lock 190, and the housing cap 450. In another embodiment, movement of the plunger 160 may correspond to movement of the plunger tip 610, wherein at least one of the plunger 160 and the plunger tip 610 may move from the interior of the housing 110 to the outside environment or at least partially above the housing cap 450. In an alternative embodiment, the plunger tip 610 may be proscribed from entering the interior of the housing 110. For example, the plunger tip 610, upon backwards movement of the scroll wheel 130, may be unable to move past the lock 190. In such an example, the inability of the plunger tip 610 to move past the lock 190 may correspond to maximum retraction of the plunger 160 facilitated by the interfacing of the post 340 with either side of the slot guide 330.

In an embodiment, the fluid dispenser 100 may progressively transition from the undrained state to the drained state. As a nonlimiting example, the fluid dispenser 100 may begin in the undrained state, wherein the cartridge 170 is full of the fluid and the plunger 160 is maximally retracted. The fluid dispenser 100 may end at the drained state, wherein the cartridge 170 is empty, and the plunger 160 is maximally extended. To transition from the undrained state to the drained state, the user may actuate the scroll wheel 130, wherein said actuation results in linear movement of the plunger 160 and the plunger tip 610. Movement of the scroll wheel 130 one predetermined increment may correspond to a predetermined distance the plunger tip 610 travels, and thus a predetermined amount of the fluid dispensed from the cartridge 170.

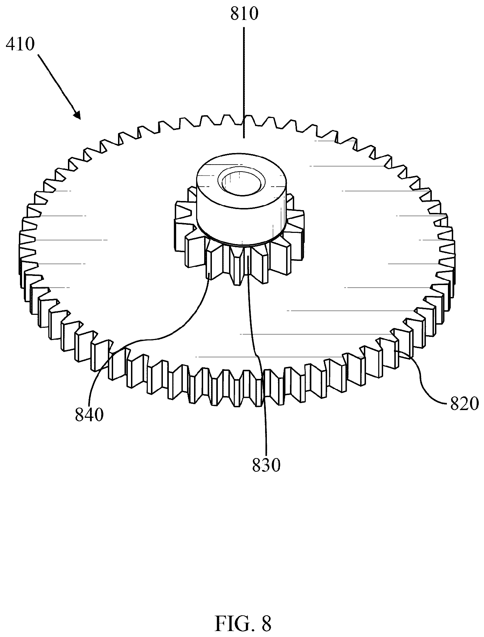

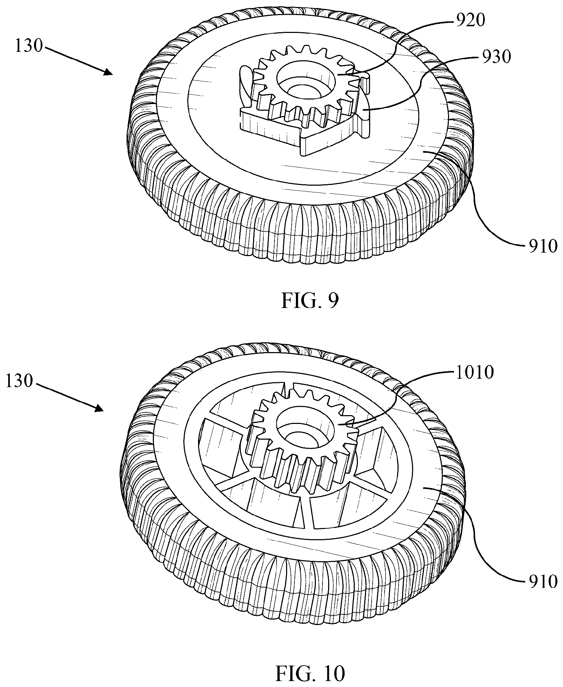

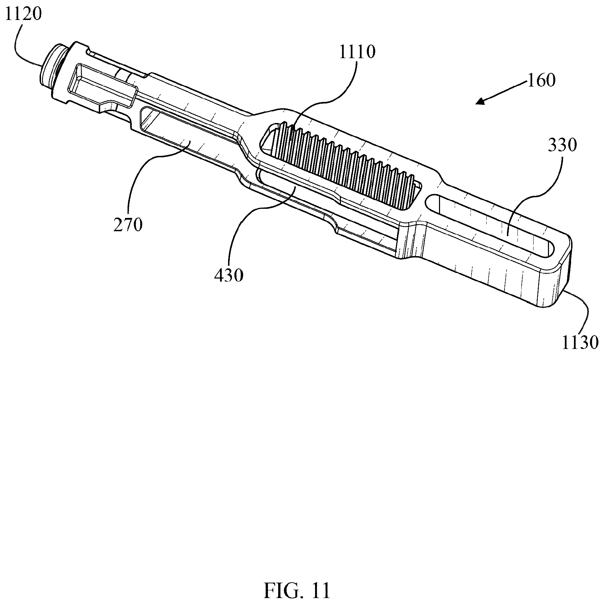

Referring to FIGS. 8-11, the one or more plunger gears 410 may be comprised of a primary gear 810 and a secondary gear 830. In an embodiment, the secondary gear 830 may be disposed atop the primary gear 810. In such an embodiment, the secondary gear 830 may have a lesser circumference than the primary gear 810, or *vice versa.*

Further, the primary gear 810 and the secondary gear 830 may be comprised of primary gear teeth 820 and secondary gear teeth 840 respectively. In an embodiment, the primary 820 and secondary 840 gear teeth may be in mechanical communication with at least one of the scroll wheel 130 and the plunger 160. For example, the primary gear 810 may be in mechanical communication with a first wheel gear 920 of the scroll wheel 130. Further, the secondary gear 830 may be in mechanical communication with a plurality of plunger teeth 1110 disposed within the gear slot 430 of the plunger 160. In an alternative embodiment, the primary gear 810, in mechanical communication with the first wheel gear 920, and the secondary gear 830, in mechanical communication with the plurality of plunger teeth 1110, may work in concert to move the plunger 160. As a nonlimiting example, rotational actuation of the scroll wheel 130 may correspond to rotational movement of the first wheel gear 910. The rotational movement of the first wheel gear 910, while in mechanical communication with the primary gear 810, may result in rotational movement of both the primary 810 and secondary 830 gears. Consequently, the rotational movement of the secondary gear 830, while in mechanical communication with the plurality of plunger teeth 1110, may result in linear movement of the plunger 160.

In an embodiment, the one or more plunger gears 410 may be comprised of a first plunger gear and a second plunger gear. The first and second plunger gears may be comprised of the primary gear 810 and secondary gear 830 respectively. In another embodiment, the first plunger gear may be disposed upon the first housing piece 210 and the second plunger gear may be disposed upon the second housing piece 220, thus forming a space where the plunger 160 may reside. In such an embodiment, the primary gear 810 of the first plunger may be in mechanical communication with the first wheel gear 910 of the scroll wheel 130, and the secondary gear 830 may be in mechanical communication with the plurality of plunger teeth 1110 disposed within the gear slot 430. Further, the primary gear 810 of the second plunger gear may be in mechanical communication with the second wheel gear 1010 of the scroll wheel 130, and the secondary gear 830 may be in mechanical communication with the plurality of plunger teeth 1110 disposed within the gear slot 430. The first plunger gear, the second plunger gear, the scroll wheel 130 and the plunger 160 may work in concert to dispense the fluid. For example, user rotational actuation of the wheel base 910 of the scroll wheel 130 may translate to rotational movement of at least one of the first wheel gear 920, the plurality of clutch actuation members 930, and the second wheel gear 1010. Moreover, the rotational movement of the first 920 and second 1010 wheel gears may translate to rotational movement of the primary 810 and secondary 830 gears comprising the first and second plunger gears. The rotational movement of the secondary gear 830, while in mechanical communication with the plurality of plunger teeth 1110, may translate to linear movement of the plunger 160 and/or the plunger tip 610. The linear movement of the plunger 160 and/or plunger tip 610 may drive the fluid from the cartridge 170, through the dispenser needle 440, and out of the cartridge tip 180 into the outside environment. The aforementioned embodiments may improve user ease of use. For example, said embodiments may comprise a gear ratio requiring less user torque to dispense the fluid. Such an embodiment may be desirable for users who have decreased hand strength. In yet a further embodiment, the gear slot 430 may be configured to house at least one of the primary gear 810 and the secondary gear 830.

In an embodiment, at least one of the first wheel gear 920, the plurality of clutch actuation members 930, and the second wheel gear 1010 may be mounted to the wheel base 910. In a further embodiment, the first wheel gear 920 and the second wheel gear 1010 may be disposed on opposite sides of the wheel base 910. In such an embodiment, the plurality of actuation members 930 may be mounted upon either the first wheel gear 920 or the second wheel gear 1010. For example, the plurality of actuation members 930 may be mounted on a first side of the wheel base 910, wherein the first wheel gear 920 or the second wheel gear 1010 may be mounted upon said actuation member 930.

The plurality of actuation members 930 may be in mechanical communication with the click clutch 420. In an embodiment, rotational movement of the scroll base 910 may translate to rotational movement of the plurality of actuation members 930. In such an embodiment, when the click clutch 420 slides off one of the plurality of actuation members 930 to a second member, the user feedback stimulus may be produced. For example, the length of one of the plurality of actuation members 930 may define the predetermined increment, wherein after the click clutch 420 slides along the length the user feedback stimulus is produced. In such an example, the distal end of the click clutch 420, in mechanical communication with the plurality of actuation members 930, may produce the auditory and haptic "click" for every length of one of the plurality of actuation members 930 the click clutch 420 travels.

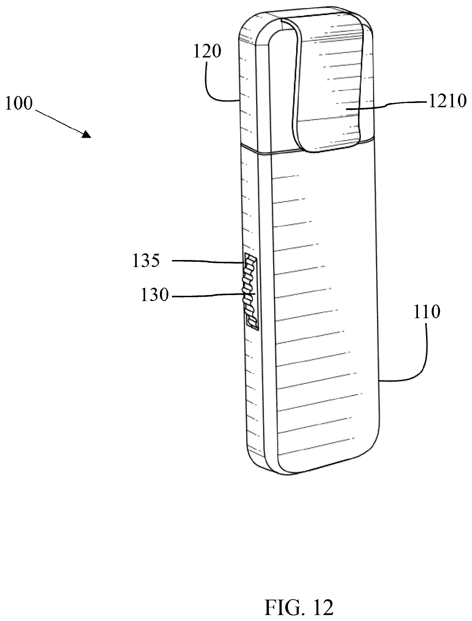

Referring to FIG. 12, the fluid dispenser 100 may be further comprised of a dispenser cap clip 1210. Said clip 1210 may be attached to the dispenser cap 120. In such an embodiment, the dispenser cap clip 1210 may allow a user to reversibly couple the fluid dispenser 100 to an article.

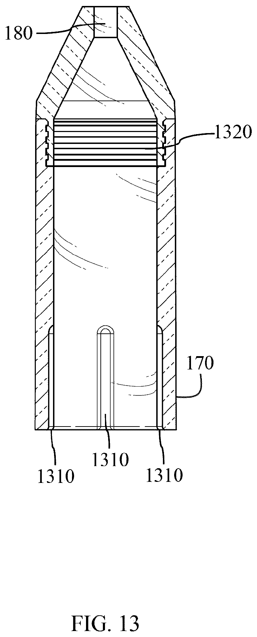

Turning to FIG. 13, the cartridge 170 may be further comprised of one or more cartridge channels 1310. In an embodiment, the one or more cartridge channels 1310 may be disposed at a first and/or second end of the cartridge 170. In another embodiment, the one or more cartridge channels 1310 may be disposed upon a cartridge end opposite of the cartridge tip 180. For example, the one or more cartridge channels may be disposed upon the first end of the cartridge 170, and the cartridge tip 180 may be disposed upon the second end of the cartridge 170, or *vice versa.*

In yet another embodiment, the one or more cartridge channels 1310 may traverse the cartridge 170, such that an interior of the cartridge 170 is in fluid communication with the outside environment. For example, the one or more cartridge channels 1310 may prevent pressure buildup within the interior of the cartridge 170 by allowing air to move freely between said interior and the outside environment. In such an example, the free movement of air between the interior of the cartridge 180 and the outside environment, facilitated via the one or more cartridge channels 1310, may prevent the fluid from leaking out of the interior of the cartridge 170.

The cartridge may be further comprised of cartridge threading 1320. In an embodiment, the cartridge threading 1320 may facilitate reverse coupling of the cartridge tip 180 to the cartridge 170. In a further embodiment, the cartridge threading 1320 may be shortened thus enabling enhanced visibility of the fluid within the cartridge 170, wherein said enhanced visibility may be achieved without assembly of the cartridge tip 180 to the cartridge 170. In further embodiments, the thread pitch and/or length of the threaded portion of the cartridge threading 1320 may be modified.

Finally, other implementations of the disclosure will be apparent to those skilled in the art from consideration of the specification and practice of the disclosure disclosed herein. It is intended that the specification and examples be considered as exemplary only, with a true scope and spirit of the disclosure being indicated by the following claims.

Various elements, which are described herein in the context of one or more embodiments, may be provided separately or in any suitable subcombination. Further, the processes described herein are not limited to the specific embodiments described. For example, the processes described herein are not limited to the specific processing order described herein and, rather, process blocks may be re-ordered, combined, removed, or performed in parallel or in serial, as necessary, to achieve the results set forth herein.

It will be further understood that various changes in the details, materials, and arrangements of the parts that have been described and illustrated herein may be made by those skilled in the art without departing from the scope of the following claims.

All references, patents and patent applications and publications that are cited or referred to in this application are incorporated in their entirety herein by reference. Finally, other implementations of the disclosure will be apparent to those skilled in the art from consideration of the specification and practice of the disclosure disclosed herein. It is intended that the specification and examples be considered as exemplary only, with a true scope and spirit of the disclosure being indicated by the following claims.

## Claims

1. A fluid dispenser comprising:
a housing;
a cartridge enclosing a fluid;
a scroll wheel comprising:
a first portion disposed within an interior of the housing,
a second portion partially protruding from the housing, and
scroll wheel interior teeth;
a first gear comprising first gear exterior teeth, the first gear disposed within the interior of the housing, the first gear exterior teeth in mechanical communication with the scroll wheel interior teeth;
a plunger comprising plunger exterior teeth in mechanical communication with the first gear exterior teeth, the plunger in fluid communication with the cartridge, the plunger further comprising a plunger slot,
wherein the scroll wheel traverses the plunger slot,
wherein the plunger is configured to move within the housing and the cartridge,
wherein the plunger is configured to compress the fluid,
wherein the compression of fluid is configured to drive the fluid from a cartridge trip disposed atop the cartridge.

2. The fluid dispenser of Claim 1, the housing comprising:
a first housing piece, having a first notch, and
a second housing piece, having a second notch,
the first notch and the second notch forming a housing aperture.

3. The fluid dispenser of either Claim 1 or Claim 2, further comprising:
a lock disposed upon the housing,
the lock in fluid communication with the interior of the housing.

4. The fluid dispenser of any preceding Claim, further comprising a dispenser cap reversibly coupled to the housing, such that the fluid dispenser may be in at least one of an open configuration and a closed configuration.

5. The fluid dispenser of any preceding Claim, wherein a rotational movement of the scroll wheel in a predetermined increment produces a feedback stimulus, preferably wherein the predetermined increment is 60 degrees.

6. The fluid dispenser of Claim 5, wherein the rotational movement of the scroll wheel in the predetermined increment compresses a predetermined volume of the fluid, preferably wherein the predetermined volume is 0.05mL.

7. The fluid dispenser of Claim 6, wherein:
the rotational movement of the scroll wheel is translated into a rotational movement of the first gear,
the rotational movement of the first gear is translated into a linear movement of the plunger, and
the linear movement of the plunger compresses the predetermined volume of the fluid.

8. A fluid dispenser comprising:
a housing;
a cartridge enclosing a fluid;
a scroll wheel comprising:
a first portion disposed within an interior of the housing,
a second portion partially protruding from the housing,
scroll wheel interior teeth,
and scroll wheel exterior teeth;
a first gear comprising first gear exterior teeth, the first gear disposed within the interior of the housing, the first gear exterior teeth in mechanical communication with the scroll wheel interior teeth;
a second gear comprising a first set of second gear teeth and a second set of second gear teeth, the second gear disposed within the interior of the housing, the first set of second gear teeth in mechanical communication with the first gear exterior teeth;
a plunger comprising plunger exterior teeth in mechanical communication with the second set of second gear teeth, the plunger in fluid communication with the cartridge, the plunger further comprising a plunger slot,
wherein the scroll wheel traverses the plunger slot,
wherein the plunger is configured to move within the housing and the cartridge,
wherein the plunger is configured to compress the fluid,
wherein the compression of fluid is configured to drive the fluid from a cartridge trip disposed atop the cartridge.

9. The fluid dispenser of Claim 8, wherein the second gear is a two-step gear.

10. The fluid dispenser of either Claim 8 or Claim 9, further comprising a roller disposed adjacent to the plunger.

11. The fluid dispenser of any one of Claims 8 to 10, further comprising a slot guide disposed within a lower portion of the plunger, preferably further comprising a post captured by the slot guide.

12. A fluid dispenser comprising:
a housing;
a cartridge enclosing a fluid;
a scroll wheel comprising:
a wheel base comprising:
a first portion disposed within an interior of the housing, and
a second portion partially protruding from the housing, and
at least one wheel gear disposed upon the wheel base;
one or more plunger gears in mechanical communication with the at least one wheel gear; and
a plunger in mechanical communication with the one or more plunger gears,
wherein the plunger is configured to move within the housing and the cartridge,
wherein the plunger is configured to compress the fluid,
wherein the compression of fluid is configured to drive the fluid from a cartridge trip disposed atop the cartridge.

13. The fluid dispenser of Claim 12, wherein the at least one wheel gear further comprises:
a first wheel gear disposed on a first side of the wheel base, and
a second wheel gear disposed on a second side of the wheel base.

14. The fluid dispenser of Claim 13, wherein the one or more plunger gears further comprise:
a first plunger gear comprising:
a first primary gear in mechanical communication with the first wheel gear, and
a first secondary gear disposed upon the first primary gear; and
a second plunger gear comprising:
a second primary gear in mechanical communication with the second wheel gear, and
a second secondary gear disposed upon the second primary gear.

15. The fluid dispenser of Claim 14, wherein the plunger further comprises:
a plurality of plunger teeth disposed within a gear slot,
the plurality of plunger teeth in mechanical communication with at least one of the first secondary gear and the second secondary gear; and
a plunger tip disposed upon a distal end of the plunger,
the plunger tip configured to compress the fluid,
the plunger tip proscribed from entering the interior of the housing, preferably
wherein:
a rotational movement of the scroll wheel is translated into a rotational movement of the first wheel gear and the second wheel gear,
the rotational movement of the first wheel gear and the second wheel gear is translated into a rotational movement of the first primary gear, the first secondary gear, the second primary gear, and the second secondary gear,
the rotational movement of the first primary gear, the first secondary gear, the second primary gear, and the second secondary gear, is translated into a linear movement of the plunger tip, and
the linear movement of the plunger tip compresses the predetermined volume of the fluid.
